# EUROPEAN PATENT SPECIFICATION

(11) **EP 2 374 498 B1**
(45) Date of publication and mention of the grant of the patent: **17.09.2014**
(21) Application number: 11152316.3
(22) Date of filing: 21.12.2007
(51) Int. Cl.: A61N 1/05

(54) **Medical implantable lead and method for manufacturing the same**
Implantierbare medizinische Leitung und Herstellungsverfahren dafür
Sonde médicale implantable et son procédé de fabrication

(30) Priority: 28.06.2007 WO PCT/SE2007/000634
(43) Date of publication of application: 12.10.2011
(62) Divisional of application: 07852147.3
(73) Proprietor: St. Jude Medical AB, 175 84 Järfälla (SE)
(72) Inventor: Hill, Rolf, SE-175 55, Järfälla (SE); Stegfeldt, Olof, SE-138 37, Älta (SE)
(74) Representative: Sjölander, Henrik

(56) References cited:
- WO-A1-01/45791
- WO-A1-99/36123
- US-A- 4 628 943
- US-A- 6 156 034
- US-A1- 2005 222 660

## Description

The invention relates to a medical implantable lead for monitoring and/or controlling of an organ inside a human or animal body, comprising two electrical conductors, each connected to a respective electrode for receiving or transmitting of electrical signals from or to the organ, and a tubular header in a distal end of the lead, wherein one electrode is positioned in the outermost distal end of the lead whereas the other is a ring formed electrode, which is mounted on the outside of the header by means of a coupling.

The invention also relates to a method for manufacturing of such a medical implantable lead.

### Background of the invention

Medical implantable leads of the above kind are well known in the art. One common field of application is for monitoring and/or controlling of the function of a human or animal heart, in which case it is inserted into the body and attached with a distal end to the heart by means of a fixating member, such as a helix which can be screwed into the tissue. A proximal end of the lead is connected to an electronic device, for performing the monitoring and/or controlling, such as a pacemaker or an implantable cardiac defibrillator.

The most distal of the electrodes can be formed as a passive electrode, in which case it is formed in the end surface of the lead and bears against the surface of the heart while being fixated to the heart wall by means of fins, tines or the like, which during implantation engages the trabecular network inside the heart and subsequently are overgrown by a layer of tissue. The distal electrode can also be of an active type, which is used both for monitoring/controlling and for fixation in that the electrode is inserted into and is engaged with the tissue, such as for example a tip provided with barbs or a helix which is screwed into the tissue. The most proximal of the electrodes is formed as a ring on the outside of the lead and positioned on a suitable distance from the distal end. The distance from the distal end is determined by the desired amplitude of the signals and an acceptable interference level.

Normally, the ring electrode is positioned at the proximal end of a so called header, which is formed as a tubular and rigid housing positioned in the distal end of the lead and adapted to carry the electrodes and the fixating member, e.g. accommodating the helix inside an inner bore of the header. The most common way to accomplish the mounting of the ring electrode to the header, is to arrange a coupling member between the header and the ring electrode which is partly inserted into the tubular bore from the proximal end of the header and partly into the ring electrode. In this way a distance is achieved between the ring electrode and the distal electrode, which is suitable for most applications and the outer surface of the ring electrode can be mounted flush with the outer surface of the header or a sleeve covering the header such that the maximum diameter of the lead can be made advantageously small. The fixation of the ring electrode in relation to the coupling member is normally achieved by an adhesive, which has to result that the assembled header, coupling member and ring electrode have to be held immovable and fixated during the time for curing of the adhesive. This is a disadvantage since it has to effect that the manufacturing process of the lead has to be suspended for a while. Moreover, this manufacturing method of the lead is also disadvantageous in that there is not provided any fixed position for the ring electrode in relation to the header. This has to effect that the position and orientation of the ring electrode in relation to the header can vary and the production yield loss of this kind of leads is comparatively high due to failures when interconnecting the ring electrode and the header.

Normally, the header as well as the coupling member are manufactured of an electrically conducting material, such as titanium, which means that a separate electrically insulating layer must be provided since the ring electrode must be electrically insulated in relation to the header. However, in prior art is also known headers made of an electrical insulating material, such as PEEK (polyetheretherketone). In this case there is no need for an electrical insulation between the ring electrode and the header. In the prior art it is also known to place the ring electrode on the outside around and bearing directly against a header of an electrically insulating material. In this way it is possible to arrange a defined position for the ring electrode on the header. However, an adhesive must be used to finally fixate the ring electrode on the header, which is a disadvantage in that the adhesive has to be applied carefully in a correct position, in order to ensure proper function of the completed lead, and requires some time for curing during which time the ring electrode may be dislodged from its position. Another disadvantage with such a design of the lead is that the ring electrode must be connected electrically, by means of a separate conductor, to a conductor in the lead. This adds additional components and manufacturing steps to the manufacturing process.

US 4,628,943 discloses a bipolar screw-in pacing lead assembly comprising an insulative tubular sheath housing two conductive members insulated from each other. A distal electrode assembly is arranged at the distal end of the tubular sheath and comprises a tissue-attachment anode electrode which is electrically and mechanically connected to the second conductive member and a ring electrode that is electrically connected to a ring cathode electrode. A proximal terminal electrode assembly is coupled to the tubular sheath and includes a cathode terminal pin coupled by a brush contact to the second conductive member and a ring electrode having a brush contact making contact with a rotatable proximal end of the first conductive member.

US 6,156,034 discloses a dual curve ablation catheter for treating atrial flutter. The ablation catheter comprises a shaft with a deflectable tip and a handle. The tip include a flexible distal segment, a stiff intermediate segment and a flexible proximal segment.

### Summary of the invention

An object of the present invention is to improve medical implantable leads according to prior art and provide a medical implantable lead which can be manufactured to a low cost. At least this object is achieved by a medical implantable lead according to claim 1.

The invention also relates to a method for manufacturing of a medical implantable lead, having essentially the same object as above. At least this object is achieved by a method according to claim 5.

According to the invention the header is manufactured of an electrically insulating material and the coupling member and the header are integrated into one unitary piece, wherein the coupling member is formed as a coupling portion in the header and formed in such a way that the ring electrode can be instantly fixated to the header by means of a quick fixating connection. Even though it is possible to also use an adhesive for increased bonding strength and fluid tight sealing of the lead, it is in this way possible to fixate the ring electrode instantly without having to wait for the adhesive to cure before proceeding with subsequent manufacturing steps.

The quick fixating connection may be formed in various ways. In two hereinafter described examples, the quick fixating connection is formed as a clamp connection, wherein the ring electrode is clamped around a coupling portion of the header. In the described examples, the clamp connection is carried out by punching locking tabs from the ring electrode, which engage in a coupling portion in form of recesses in the outer surface of the header and lock the ring electrode in relation to the header. This kind of clamp connection is advantageous in so far as it prevents the ring electrode from being displaced in the longitudinal direction as well being rotated in relation to the header. However, the clamp connection can be carried out also in other ways, such as a clamp connection where the circumference of the ring electrode is crimped around and pressed towards the header. A quick fixating connection in form of a clamp connection is also advantageous in so far as it easily can be used to locate the ring electrode with different desirable distance from the distal tip of the medical implantable lead, as is illustrated in two different examples hereinafter.

In the described and illustrated clamp connection examples, the ring electrode is formed such that a proximal sleeve portion, in the mounted state, reaches over an electrical conductor, which suitably is in form of an outer wire coil and which provides electrical connection to the proximal end of the medical implantable lead, such that the ring electrode also can be electrically connected to the outer wire coil by means of a clamp connection. In this way no separate connector is required for connection of the ring electrode to the outer wire coil. Preferably, the clamp connection of the ring electrode to the header as well as to the outer wire coil, is performed in the same operation. Also preferably, the connection of the outer wire coil to the header is performed in the same operation and with the same clamp connection as when connecting the ring electrode to the outer wire coil. However, it is to be understood that it would also be possible to perform the clamp connection of the ring electrode to the header and to the outer wire coil in separate operations and the outer wire coil can be attached to the header by some other means, such as by welding, for increased strength.

In an hereinafter described and illustrated embodiment of the invention, the quick fixating connection is formed as a snap fit attachment, such that the coupling portion in the proximal end of the header is provided with a circumferential groove on the outside, into which an inner, circumferential bead in a distal end of the ring electrode can go into engagement by deformation of the material in the ring electrode.

However, the snap fit attachment could be formed in many other ways and it would also be possible to arrange a quick fixating connection in form of a thermo connection between the ring electrode and the header.

In the described and illustrated embodiments of the invention, the fixating member for fixating the lead to an organ inside a body, is formed as a helix, which is accommodated inside a bore in the header and which can be screwed out from the distal end and into the tissue of an organ. It is to be understood, however, that the fixating member can be formed in many other ways, such as for example as a sharp tip provided with barbs to be penetrated into the tissue or as fins or tines to be engaging the trabecular network inside a heart and to be subsequently overgrown by tissue. The lead can also be adapted for monitoring and/or controlling of other organs than a heart inside a body.

### Brief description of the drawings

A detailed description of a prior art embodiment as well as embodiments according to the invention, will hereinafter be given by way of example with reference to the drawings, in which:
- Fig 1: is a longitudinal section through a distal end of a lead according to a prior art embodiment;
- Fig 2: is a longitudinal section through a distal end of a lead according to a first embodiment of the invention;
- Fig 3: is an enlarged detail in longitudinal section of the connection between the ring electrode and the header, according to the first embodiment, in a disassembled state;
- Fig 4: is a partly exploded perspective view of the lead according to fig 3;
- Fig 5: is a longitudinal section through a distal end of a lead according to a first example in an unclamped state;
- Fig 6: is a longitudinal section according to fig 6 during clamping;
- Fig 7: is a longitudinal section through a completed lead according to figs 6 and 7; and
- Fig 8: is a longitudinal section through a lead according to a second example.

### Detailed description of a prior art embodiment and embodiments according to the invention

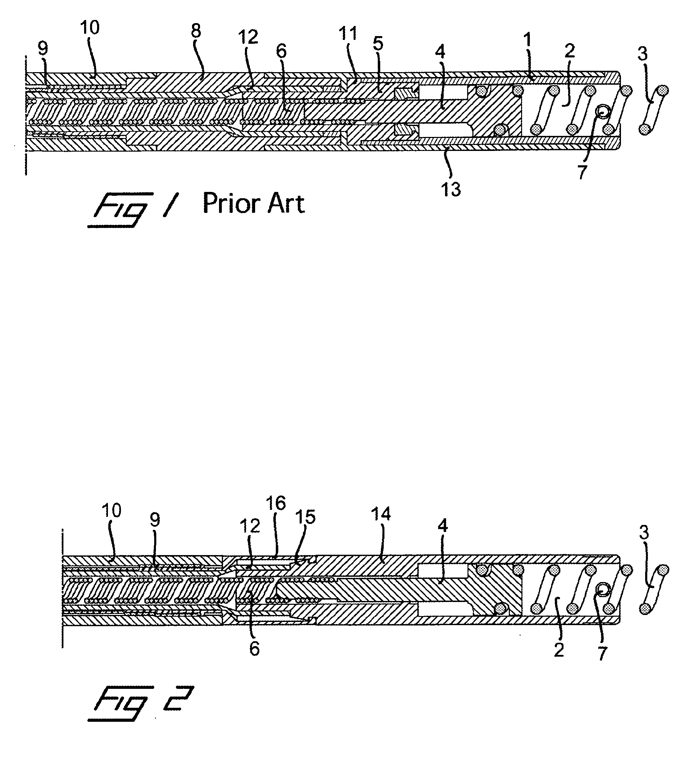

Reference is first made to fig 1 of the drawings, in which is illustrated a prior art embodiment of a distal end of a medical implantable lead. In the most distal end the lead comprises a tubular header 1 of an electrically conducting material such as titanium. In a distal bore 2 of the header, a helix 3 is accommodated, which is illustrated in a partially projecting state. The helix is mounted on a distal end of a shaft 4, which in its turn is journalled in a coupling member 5 such that it can be rotated as well as displaced in a longitudinal direction in relation to the coupling. The rotation of the helix 3 is actuated from the proximal end of the lead by rotation of an inner wire coil 6, which also functions as an electrical conductor from the proximal to the distal end. The helix 3 is in engagement with a post 7 on the inside of the header 1, such that when the inner wire coil 6 is rotated, the helix will be screwed out and advanced forward out from the header, which will have to effect that also the shaft 4 and the inner wire coil 6 will be advanced forward. When implanting the lead into a body, the helix is screwed out and into an organ such that the distal end is attached to the organ and the helix will function as an electrode in the organ. A second electrode is arranged in the proximal end of the header in form of a ring electrode 8, which is in electrical contact with the proximal end by means of an outer wire coil 9. Outside of the outer wire coil, an outer sleeve 10 is applied for protection of the lead.

Besides the function of rotationally bearing the shaft 4, the coupling member 5 also has the function of mounting and fixating the ring electrode 8. For this purpose, the coupling member 5 is formed with an intermediary section 11 having a comparatively large diameter, namely a diameter being equal to the outer diameter of the header 1, and end portions having a comparatively small diameter. When assembling the lead, one of the end portions of member is metallic. The other of the end portions is utilized for bearing of the ring electrode 8. However, since the header 1 and the coupling member 5 are metallic, they will have the same electrical potential as the shaft 4 and the helix 3. Accordingly, the ring electrode must be electrically insulated from the coupling member 5, which will have a different electrical potential. This is achieved by means of an inner sleeve 12, of silicone or the like, which surrounds the inner wire coil 6 and is extended up over a proximal end portion of the coupling member. The ring electrode 8 is positioned over the inner sleeve 12 and will accordingly be electrically insulated in relation to the coupling member 5, the header 1, the shaft 4 and the helix 3. However, the distal end of the ring electrode, is not allowed to come into electrical contact with the proximal end of the large diameter portion of the coupling member. Accordingly, when fixating the ring electrode to the coupling member by means of an adhesive, the ring electrode has to be held immovable, preferably in a fixture, in relation to the coupling member together with the header, for as long time as until the adhesive has been cured and the ring electrode is fixated. This means that the assembling of the lead has to be suspended for a while which is a disadvantage for the work flow when assembling the lead. Moreover, since there is no fixed position for the ring electrode on the coupling member, it frequently happens that the distance between the distal end of the ring electrode and the proximal end of the large diameter portion of the coupling becomes too small, in which case there is a risk for shortcircuiting, or too large in which case it is a risk that the assembled header, coupling and ring electrode does not fit with the rest of the lead components. It also happens that the header and the ring electrode becomes misaligned in relation to each other or that the insulating inner sleeve becomes damaged when passing the distal end of the ring electrode over the coupling member and the inner sleeve. All of these situations may lead to a rejection of the lead. Moreover, since the header is electrically conductive and has the same electrical potential as the distal electrode, i.e. the helix, it has to be covered by an electrically insulating layer in form of a header sleeve 13, of e.g. silicone, which normally is bonded directly onto the header such that the adhesive also penetrates into is bonded directly onto the header such that the adhesive also penetrates into the gap between the proximal flange of the ring electrode and the large diameter portion 11 of the coupling member.

As is evident from the above description, the distal end of a medical implantable lead according to prior art, is composed of many different components and several different assembling steps are required for the manufacturing. Accordingly, it is desirable to provide a medical implantable lead which contains less component parts and requires fewer assembling steps for manufacturing, in order to reduce costs.

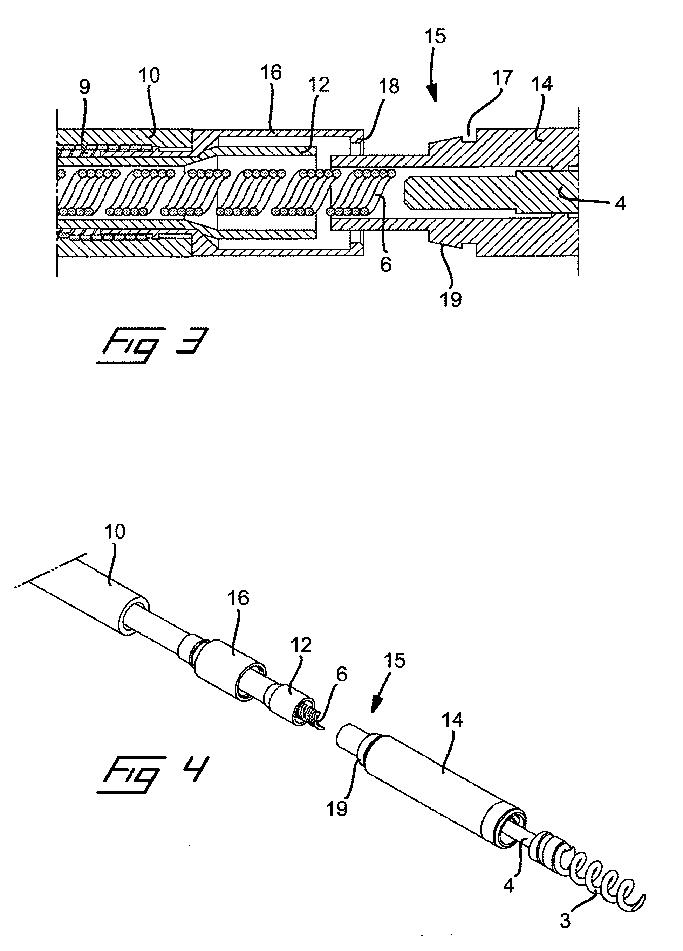

Now reference is made to figs 2-4 for description of an embodiment of a medical implantable lead according to the invention. Here the coupling member and the header are integrated into one unitary piece and made of an electrically insulating material, e.g. of PEEK, in form of a header 14 having a coupling portion 15 in the proximal end. Moreover, the proximal coupling portion is formed with a quick fixating connection, which allows for an instant connection between the coupling portion and a properly adapted ring electrode 16. More precisely, the coupling portion of the header is formed with a circumferential groove 17 in its outer surface, whereas the ring electrode is formed with an inner, circumferential bead 18 in its distal end, such that the ring electrode can be snap-fit connected to the header. To facilitate the snap-fit engagement, the header is formed with an inclined surface 19 proximal to the groove 17, such that the bead 18 of the ring electrode, upon pressing the ring electrode and the header axially towards each other, may slide over the inclined surface 19, while slightly deforming the ring electrode, and snap into the groove.

The header 14 according to the invention will accordingly serve several purposes. On the one hand it will accommodate the helix inside the header bore 2 such that the helix may be completely contained inside the bore during inserting of the lead into the body and may be screwed out from the distal end and into the tissue for fixating the lead to an organ. Moreover, the header will provide for bearing of the shaft 4 and allow rotation as well as longitudinal displacement of the shaft. The header also provides, as already described, for means for quick fixating of the ring electrode to the header. Finally, the header provides for electrical insulation of the inner wire coil 6, shaft 4 and helix 3 from the ring electrode 16 and the outer wire coil 9. Since the ring electrode 16 is supported by the header 14 and the inner sleeve 12, which is extended over the outside of a proximal portion of the header, no additional insulating components are required between these parts.

Accordingly, a medical implantable lead according to the invention, will facilitate assembling of the lead and reduces the number of components required. For example, no separate coupling member is required and since the header is manufactured of an electrically insulating material, no additional insulating layer, as the layer 13 in fig 1, is required on the outside of the header. Moreover, the lead can be manufactured with an increased precision which reduces production yield losses. All these factors allows for manufacturing of medical implantable leads to a lower cost.

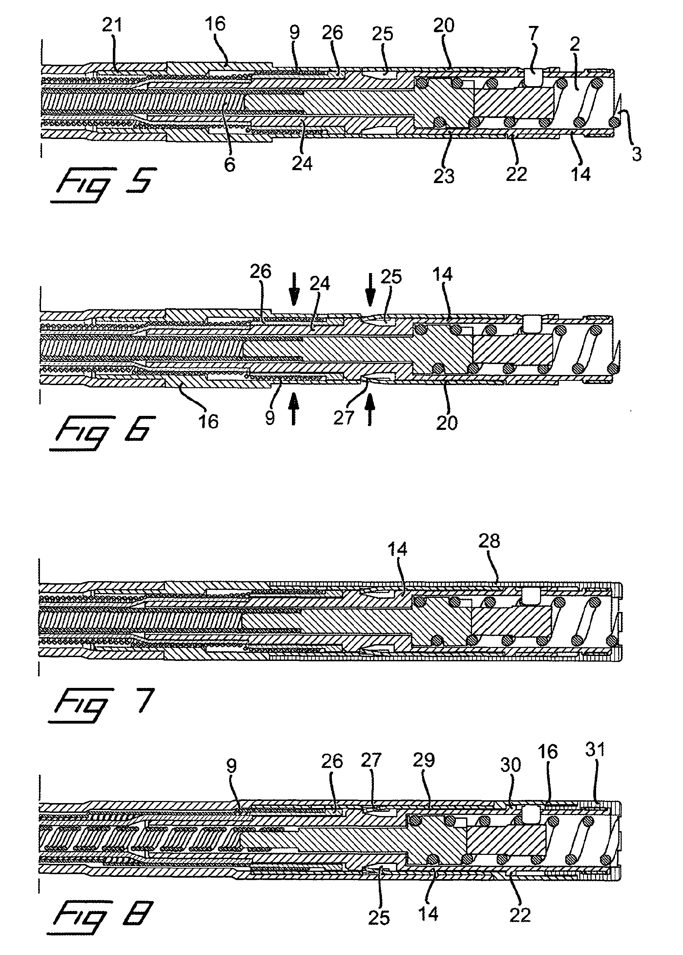

Reference is then made to figs 5 to 7, in which are illustrated a first example of a medical implantable lead. In this example the attachment between the header sleeve 14, being made of an electrically insulating material, and the ring electrode 16, is performed by means of a clamping connection. More precisely, the ring electrode 16 is provided with a distal sleeve portion 20 and a proximal sleeve portion 21 having slightly smaller outer diameters than the actual ring electrode 16. The header sleeve 14 in its turn, comprises an enlarged diameter portion 22 near its distal end, an intermediary support portion 23 for the distal sleeve portion 20 of the ring electrode, and a proximal support portion 24 with reduced diameter for the outer wire coil 9. The intermediary support portion 23 is formed with a coupling portion in form of recesses 25 around its periphery. When mounting the ring electrode 16 onto the header sleeve 14, the ring electrode 16 is slid onto the header sleeve 14 from its proximal end, until the end of the distal sleeve portion 20 abuts a proximal end surface of the enlarged diameter portion 22 of the header sleeve. Subsequently, the outer wire coil 9 together with a crimping sleeve 26 is inserted between the inner periphery surface of the ring electrode 16 and the proximal support portion 24 of the header sleeve until the distal end of the crimping sleeve 26 abuts a proximal end of the intermediary support portion 23. Thereafter, the assembly is positioned in a clamping tool by means of which clamping forces are applied over the recesses 25 and the proximal support portion 24, as is illustrated by the arrows in fig 6. This has to effect that locking tabs 27 will be punched out from the distal sleeve portion 20 of the ring electrode 16, which will be bent down into the recesses 25 and lock the ring electrode in relation to the header sleeve, and a proximal portion of the distal sleeve portion 20 will be pressed and crimped around the proximal support portion 24 such that the outer wire coil 9 and the clamping sleeve 26 will be pressed towards and secured in relation to the proximal support portion 24 by means of the clamp connection of the ring electrode 16. Finally, an outer protective sleeve 28 of an electrically insulating material, is arranged over the distal end of the header sleeve 14 and the distal sleeve portion 20 of the ring electrode, as is illustrated in fig 7.

In fig 8 is illustrated a second example which, similar to the example illustrated in figs 5 to 7, is formed with a quick fixating connection in form of a clamping connection. In this example, however, the electrode surface of the ring electrode 16 is positioned close to the distal end of the header sleeve. This is accomplished by forming the ring electrode 16 with a proximal sleeve portion 29 which, at a transition section to the electrode surface, is formed with a stop surface 30 facing towards the distal end, which abuts against an enlarged diameter portion 22 of the header sleeve 14. The raised portion forming the electrode surface of the ring electrode 16, rests partly on the enlarged diameter portion 22 and partly on a silicone cap 31 in the end of the header sleeve. In all other respects, this example is similar to the example relating to the figs 5 and 6, i.e. the header sleeve is provided with recesses 25 in the header for forming of locking tabs 27 in the ring electrode and the outer wire coil 9 is attached to the header sleeve by clamping the ring electrode around it and a clamping sleeve 26.

## Claims

1. A medical implantable lead for monitoring and/or controlling of an organ inside a human or animal body, comprising two electrical conductors (6, 9), each connected to a respective electrode (3, 16) for receiving or transmitting of electrical signals from or to the organ, and a tubular header (14) in a distal end of the lead, wherein one electrode (3) is positioned in the outermost distal end of the lead whereas the other is a ring formed electrode (16), which is mounted on the outside of the header (14) by means of a coupling, whereby the coupling and the header (14) are integrated into one unitary piece of an electrically insulating material such that the header (14) is formed with a coupling portion (15), **characterized in that** the coupling portion (15) is adapted for a quick fixating connection of the ring electrode (16) to the header (14) and the quick fixating connection is a snap fit connection.

2. A medical implantable lead according to claim **1, characterized in that** the snap fit connection comprises an outer, circumferential groove (17) in the proximal end of the header (14) and an inner, circumferential bead (18) in the distal end of the ring electrode (16).

3. A medical implantable lead according to claim 2, **characterize d** in that the header (14) is formed with an inclined surface (19) proximal to the groove (17), the inclined surface (19) is configured to cause, upon pressing the ring electrode (16) and the header (14) axially towards each other, the bead (18) of the ring electrode (16) to slide over the inclined surface (19).

4. A medical implantable lead according to any of the claims 1 to 3, **characterized in that** besides the quick fixating connection, an adhesive is used for increased bonding strength and fluid sealing.

5. A method for manufacturing of a medical implantable lead of the type adapted for monitoring and/or controlling of an organ inside a human or animal body and comprising two electrical conductors (6, 9), each connected to a respective electrode (3, 16) for receiving or transmitting of electrical signals from or to the organ, and a tubular header (14) in a distal end of the lead, wherein one electrode (3) is positioned in the outermost distal end of the lead whereas the other is a ring formed electrode (16), which is mounted on the outside of the header by means of a coupling, comprising the steps of:
integrating the coupling and the header (14) into one unitary piece of an electrically insulating material such that the header (14) is formed with a coupling portion (15);
forming the coupling portion such that it is adapted for a quick fixating connection of the ring electrode (16) to the header;
forming the ring electrode (16) with a mating engagement means (18) for engagement with the coupling portion, and
forming the quick fixating connection as a snap fit connection.

6. A method according to claim 5, comprising the further step of using an adhesive between the header (14) and the ring electrode (16) for achieving an increased bonding strength and fluid tight seal.

7. A method according to claim 5 or 6, wherein the step of forming the coupling portion (15) comprises forming the coupling portion such that it comprises a circumferential groove (17) in its outer surface and wherein the step of forming the ring electrode (16) with a mating engagement means comprises forming the ring electrode with an inner circumferential bead (18) in its distal end such that a snap fit connection of the ring electrode to the header is enabled.

## Patentansprüche

1. Implantierbare medizinische Leitung zum Überwachen und/oder Steuern eines Organs innerhalb eines menschlichen oder tierischen Körpers, umfassend zwei elektrische Leiter (6, 9), die jeweils mit einer jeweiligen Elektrode (3, 16) zum Aufnehmen oder Übertragen elektrischer Signale von oder zu dem Organ verbunden sind, und ein röhrenförmiges Kopfteil (14) in einem distalen Ende der Leitung, wobei eine Elektrode (3) in dem äußersten distalen Ende der Leitung positioniert ist, wohingegen die andere eine ringförmige Elektrode (16) ist, die an der Außenseite des Kopfteils (14) mittels einer Kupplung angebracht ist, wobei die Kupplung und das Kopfteil (14) in ein einheitliches Stück eines elektrisch isolierenden Materials integriert sind, derart, dass das Kopfteil (14) mit einem Kupplungsabschnitt (15) gebildet ist, **dadurch gekennzeichnet, dass** der Kupplungsabschnitt (15) für eine Schnellbefestigungsverbindung der Ringelektrode (16) an dem Kopfteil (14) ausgelegt ist und die Schnellbefestigungsverbindung eine Einrastverbindung ist.

2. Implantierbare medizinische Leitung nach Anspruch 1, **dadurch gekennzeichnet, dass** die Einrastverbindung eine äußere Umfangsnut (17) in dem proximalen Ende des Kopfteils (14) und einen inneren Umfangsflansch (18) in dem distalen Ende der Ringelektrode (16) umfasst.

3. Implantierbare medizinische Leitung nach Anspruch 2, **dadurch gekennzeichnet, dass** das Kopfteil (14) mit einer schrägen Fläche (19) nahe der Nut (17) gebildet ist, wobei die schräge Fläche (19) ausgelegt ist, auf ein Drücken der Ringelektrode (16) und des Kopfteils (14) axial aufeinander zu dazu zu führen, dass der Flansch (18) der Ringelektrode (16) über die schräge Fläche (19) gleitet.

4. Implantierbare medizinische Leitung nach einem der Ansprüche 1 bis 3, **dadurch gekennzeichnet, dass** neben der Schnellbefestigungsverbindung ein Kleber zum Erhöhen einer Bondierungsfestigkeit und für eine Flüssigkeitsdichtung verwendet wird.

5. Verfahren zum Herstellen einer implantierbaren medizinischen Leitung des Typs, der ausgelegt ist zum Überwachen und/oder Steuern eines Organs innerhalb eines menschlichen oder tierischen Körpers, und umfassend zwei elektrische Leiter (6, 9), die jeweils mit einer jeweiligen Elektrode (3, 16) zum Aufnehmen oder Übertragen elektrischer Signale von und zu dem Organ verbunden sind, und ein röhrenförmiges Kopfteil (14) in einem distalen Ende der Leitung, wobei eine Elektrode (3) in dem äußersten distalen Ende der Leitung positioniert ist, wohingegen die andere eine ringförmige Elektrode (16) ist, die an der Außenseite des Kopfteils mittels einer Kupplung befestigt ist, umfassend die Schritte:
Integrieren der Kupplung und des Kopfteils (14) in ein einheitliches Stück eines elektrisch isolierenden Materials, derart, dass das Kopfteil (14) mit einem Kupplungsabschnitt (15) gebildet wird;
Bilden des Kupplungsabschnitts derart, dass dieser ausgelegt ist für eine Schnellbefestigungsverbindung der Ringelektrode (16) mit dem Kopfteil;
Bilden der Ringelektrode (16) mit einer ineinander greifenden Eingriffseinrichtung (18) zum Eingriff in den Kupplungsabschnitt, und
Bilden der Schnellbefestigungsverbindung als eine Einrastverbindung.

6. Verfahren nach Anspruch 5, umfassend den weiteren Schritt eines Verwendens eines Klebers zwischen dem Kopfteil (14) und der Ringelektrode (16) zum Erreichen einer erhöhten Bondierungsfestigkeit und einer flüssigkeitsfesten Dichtung.

7. Verfahren nach Anspruch 5 oder 6, wobei der Schritt eines Bildens des Kupplungsabschnitts (15) ein derartiges Bilden des Kupplungsabschnitts umfasst, dass er eine Umfangsnut (17) in seiner äußeren Fläche umfasst, und wobei der Schritt eines Bildens der Ringelektrode (16) mit einer ineinander greifenden Eingriffseinrichtung ein Bilden der Ringelektrode mit einem inneren Umfangsflansch (18) in ihrem distalen Ende umfasst, derart, dass eine Einrastverbindung der Ringelektrode mit dem Kopfteil ermöglicht wird.

## Revendications

1. Sonde médicale implantable pour la surveillance et/ou le contrôle d'un organe dans un corps humain ou animal, comprenant deux conducteurs électriques (6, 9), chacun connecté à une électrode respective (3, 16) pour recevoir ou transmettre des signaux électriques en provenance ou à destination de l'organe, et une tête tubulaire (14) à une extrémité distale de la sonde, une électrode (3) étant positionnée à l'extrémité distale la plus extérieure de la sonde, tandis que l'autre est une électrode en forme d'anneau (16) qui est montée sur l'extérieur de la tête (14) au moyen d'un couplage, le couplage et la tête (14) étant ainsi intégrés en une pièce unitaire d'un matériau électriquement isolant de manière que la tête (14) soit formée avec une partie de couplage (15), **caractérisée en ce que** la partie de couplage (15) est adaptée à une connexion de fixation rapide de l'électrode annulaire (16) à la tête et **en ce que** la connexion de fixation rapide est une connexion à pression.

2. Sonde médicale implantable selon la revendication 1, **caractérisée en ce que** la connexion à pression comprend une rainure circonférentielle extérieure (17) à l'extrémité proximale de la tête (14) et un bourrelet circonférentiel intérieur (18) à l'extrémité distale de l'électrode annulaire (16).

3. Sonde médicale implantable selon la revendication 2, **caractérisée en ce que** la tête (14) est formée avec une surface inclinée (19) proximale à la rainure (17), la surface inclinée (19) étant configurée pour provoquer, lors d'une pression de l'électrode annulaire (16) et de la tête (14) axialement l'une vers l'autre, un glissement du bourrelet (18) de l'électrode annulaire (16) sur la surface inclinée (19).

4. Sonde médicale implantable selon l'une quelconque des revendications 1 à 3, **caractérisée en ce qu'**en plus de la connexion de fixation rapide, un adhésif est utilisé pour augmenter la force d'adhérence et l'étanchéité aux fluides.

5. Procédé de fabrication d'une sonde médicale implantable du type conçu pour surveiller et/ou contrôler un organe dans un corps humain ou animal, et comprenant deux conducteurs électriques (6, 9), chacun connecté à une électrode respective (3, 16) pour recevoir ou transmettre des signaux électriques en provenance ou à destination de l'organe, et une tête tubulaire (14) à une extrémité distale de la sonde, une électrode (3) étant positionnée à l'extrémité distale la plus extérieure de la sonde, tandis que l'autre est une électrode en forme d'anneau (16) qui est montée sur l'extérieur de la tête au moyen d'un couplage, comprenant les étapes suivantes :
l'intégration du couplage et de la tête (14) en une pièce unitaire d'un matériau électriquement isolant de manière que la tête (14) soit formée avec une partie de couplage (15) ;
la formation de la partie de couplage de manière qu'elle soit adaptée à une connexion de fixation rapide de l'électrode annulaire (16) à la tête ;
la formation de l'électrode annulaire (16) avec un moyen d'entrée en prise conjugué (18) pour l'entrée en prise avec la partie de couplage, et
la formation de la connexion de fixation rapide sous la forme d'une connexion à pression.

6. Procédé selon la revendication 5, comprenant l'étape supplémentaire d'utilisation d'un adhésif entre la tête (14) et l'électrode annulaire (16) pour obtenir une force d'adhérence et une étanchéité aux fluides accrues.

7. Procédé selon la revendication 5 ou 6, dans lequel l'étape de formation de la partie de couplage (15) comprend la formation de la partie de couplage de manière qu'elle comprenne une rainure circonférentielle (17) dans sa surface extérieure et dans lequel l'étape de formation de l'électrode annulaire (16) avec un moyen d'entrée en prise conjugué comprend la formation de l'électrode annulaire avec un bourrelet circonférentiel intérieur (18) à son extrémité distale de manière à permettre une connexion à pression de l'électrode annulaire à la tête.
